# EUROPEAN PATENT SPECIFICATION

(11) **EP 1 513 471 B1**
(45) Date of publication and mention of the grant of the patent: **06.12.2006**
(21) Application number: 03755337.7
(22) Date of filing: 30.04.2003
(51) Int. Cl.: A61F 2/06

(54) **ENDOLUMINAL DEVICE HAVING BARB ASSEMBLY**
ENDOLUMINALE VORRICHTUNG MIT EINER VERANKERUNGSEINHEIT
DISPOSITIF INTRALUMINAL COMPORTANT UN ENSEMBLE DE BARBES

(30) Priority: 22.05.2002 US 153351
(43) Date of publication of application: 16.03.2005
(73) Proprietor: Boston Scientific Limited, St. Michael (BB)
(72) Inventor: MINASIAN, Zarouhi, Bedford, MA 01730 (US); WELDON, James, Roslindale, MA 02131 (US)
(74) Representative: Schwabe - Sandmair - Marx
(86) International application number: PCT/US2003/013533
(87) International publication number: WO 2003/099167

(56) References cited:
- EP-A- 0 974 314
- WO-A-00/66031
- WO-A-01/35864
- US-A- 5 344 427

## Description

This invention relates generally to endoluminal devices, and more particularly concerns implants such as stents and grafts for placement in an area of a body lumen that has been weakened by damage or disease, such as by aneurysms of the abdominal aorta. In particular, the present invention relates to such devices having barbs that engage the body lumen upon or after deployment of the device.

### BACKGROUND OF THE INVENTION

A stent is an elongated device used to support an intraluminal wall. In the case of a stenosis, a stent provides an unobstructed conduit through a body lumen in the area of the stenosis. Such a stent may also have a prosthetic graft layer of fabric or covering lining the inside and/or outside thereof. A covered stent is commonly referred to in the art as an intraluminal prosthesis, an endoluminal or endovascular graft (EVG), an endoluminal device, or a stent-graft. As used herein, the term "implant" shall mean any covered stent or uncovered stent or other medical device suitable for implantation in a body and for use in connection with the present invention.

A stent-graft may be used, for example, to treat a vascular aneurysm by removing the pressure on a weakened part of an artery so as to reduce the risk of rupture. Typically, a stent is implanted in a blood vessel at the site of a stenosis or aneurysm endoluminally, i.e. by so-called "minimally invasive techniques" in which the stent, restrained in a radially compressed configuration by a sheath or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means. The term "proximal" as used herein refers to portions of the stent or delivery system relatively closer to the end outside of the body, whereas the term "distal" is used to refer to portions relatively closer to the end inside the body.

When the introducer has been threaded into the body lumen to the stent deployment location, the introducer is manipulated to cause the stent to,be ejected from the surrounding sheath or catheter in which it is restrained (or alternatively the surrounding sheath or catheter is retracted from the stent), whereupon the stent expands to a predetermined diameter at the deployment location, and the introducer is withdrawn. Stent expansion may be effected by spring elasticity, balloon expansion, or by the self-expansion of a thermally or stress-induced return of a memory material to a preconditioned expanded configuration.

Among the many applications for stent-grafts is that of deployment in lumen for repair of aneurysms, such as abdominal aortic aneurysms (AAA). An AAA is an area of increased aortic diameter that generally extends from just below the renal arteries to the aortic bifurcation. AAA generally results from deterioration of the arterial wall, causing a decrease in the structural and elastic properties of the artery. In addition to a loss of elasticity, this deterioration also causes a slow and continuous dilation of the lumen.

The standard surgical repair of AAA is an extensive and invasive procedure typically requiring a weeklong hospital stay and an extended recovery period. To avoid the complications of the surgical procedure, practitioners commonly resort to a minimally invasive procedure using endoluminal stent-grafts to reinforce the weakened vessel wall, as mentioned above. At the site of the aneurysm, the practitioner deploys the stent-graft, anchoring it above and below the aneurysm to relatively healthy tissue. The anchored stent-graft diverts blood flow away from the weakened arterial wall, minimizing the exposure of the aneurysm to high pressure.

Intraluminal stents for repairing a damaged or diseased artery or to be used in conjunction with a graft for delivery to an area of a body lumen that has been weakened by disease or damaged, such as an aneurysm of the abdominal aorta, are well established in the art of medical science. The use and description of such intraluminal stents are set forth in U.S. Patent Nos. 5,681,346; 5,800,526; and 5,843,164. These references are each incorporated in their entirety as part of this specification. One aspect of the use of such intraluminal stents are the means by which such devices are secured within the intraluminal body in which they are to be deployed. This is important because subsequent movement of the stent (or "migration") could cause the aneurysm to become exposed to blood pressure. In particular, if the device migrates proximally over time, a leak at the distal end of the device (i.e., a "type I endoleak") could cause blood to undesirably flow to the aneurysm.

Stents with fixed barbs have been used to engage the vessel wall as the deployment sheath is pulled back from the stent. However, such stents with fixed integrated barbs are difficult to load into the catheter deployment system. Fixed barbs are not flush to the perimeter of the stent and therefore have a tendency to prevent the stent from being loaded or to cause the stent to become lodged inside the catheter during loading. Moreover, catheter deployment systems used to deploy stents with barbs are commonly scratched during the deployment of the stent. Scratching of the catheter deployment system can cause plastic particulate from the catheter deployment system to enter the bloodstream, potentially forming an embolus.

WO 01/35864, which is considered to represent the closest prior art, discloses a multi-section filamentary stent which comprises a braided section, which is a cylindrical mesh of a first set of filaments, connected to at least one wound section comprising a second set of one or more filaments having a repeating configuration with a bent portion. The two sections are preferably connected by at least one continuous filament extending into both sections. The two sections may be connected by a weld, a suture, a common graft, an overlapping portion of the two sections, or one or more filaments of one section looping through portions of the other section. The stent may comprise a first section, having a braided first stent architecture with a first flexibility and a first radial force, and a second section, having a non-braided second stent architecture with a second flexibility less than the first flexibility and a second radial force greater than the first radial force, in which at least one continuous filament is integral to both the first and second sections. The stent may also comprise one or more filaments in a repeating configuration having at least one bent portion, wherein the repeating configuration defines at least a first hoop comprising hexagonal cells, the first hoop axially adjacent to a second hoop that does not comprise hexagonal cells. The stent may also comprise a plurality of zig-zag or sinusoidal members wherein at least a first zig-zag member is overlaid upon a second zig-zag member and out of phase with the first zig-zag member to form an overlap between at least one strut of the first zig-zag member and one strut of the second zig-zag member

Accordingly, it can be seen that while the art has advanced the use of barbs to minimize migration of a deployed stent-graft, such barbs bring with them additional or new problems such as damaging the wall of the vessel or hindering the placement of the stent and body graft. While the art has attempted to address such problems, there still remains a need for improvement in the art. Such improvement is critical inasmuch as scratching of the deployment system can cause plastic or other particulate from the deployment system to enter the blood stream, potentially forming an embolus.

### SUMMARY OF THE INVENTION

In view of its purposes and the needs of the prior art, the present invention provides an endoluminal device comprising an implant and a barb or barb assembly. According to a first embodiment, a device for implantation in a body lumen comprises an implant and at least one barb assembly. The implant may be a stent having a radially compressed configuration and a radially expanded configuration and comprising at least one filament which pivots as the stent moves between the radially compressed configuration and the radially expanded configuration. The barb assembly comprises: (i) a first portion attached to the stent, (ii) a bend, and (iii) a second portion, disposed opposite the first portion relative to the bend and having a bearing surface. The second portion is adapted to protrude radially inward when the stent is in the radially compressed configuration. The filament radially contacts and imparts a radially outward force against the bearing surface as the stent moves from the radially compressed configuration to the radially expanded configuration to cause the second portion to protrude radially outward (or "flip" outwardly) when the implant, preferably a stent is in its radially expanded configuration .

The foregoing general description and subsequent detailed description are representative, not restrictive, of the invention.

### BRIEF DESCRIPTION OF THE DRAWINGS

The invention is best understood when the following detailed description is read with reference to the attached drawing, in which:
Fig. 1 depicts a view of a portion of an endoluminal device according to a first embodiment of the present invention;
Fig. 2 depicts an enlarged portion of the device shown in Fig. 1 and shows a barb assembly according to the present invention;
Fig. 3a depicts a perspective view of a portion of the device shown in Fig. 1 in its radially expanded configuration and shows a barb assembly according to the first embodiment of the present invention;
Fig. 3b depicts a perspective view of a portion of the device shown in Fig. 1 in its radially compressed configuration and shows a barb assembly according to the first embodiment of the present invention;

### DETAILED DESCRIPTION OF THE INVENTION

The invention will next be illustrated with reference to the figures wherein the same numbers indicate similar elements in all figures. Such figures are intended to be illustrative rather than limiting and are included herewith to facilitate the explanation of the apparatus of the present invention.

The present invention is directed to devices for implantation in a body lumen. Such devices include an endoluminal device used to treat an Abdominal Aortic Aneurysm (AAA). Such an endoluminal device typically comprises a stent having a graft extending along a portion of the stent. Devices according to the present invention may also include other implants which have a stent-like structure and, after implantation of which, migration is sought to be minimized. The body lumen in which a device of the present invention may by implanted include any body lumen in which such devices are typically implanted to perform a wide range of medical functions. In the AAA application, the body lumen is at least one artery, such as the aorta or the aorta and one or both iliac arteries.

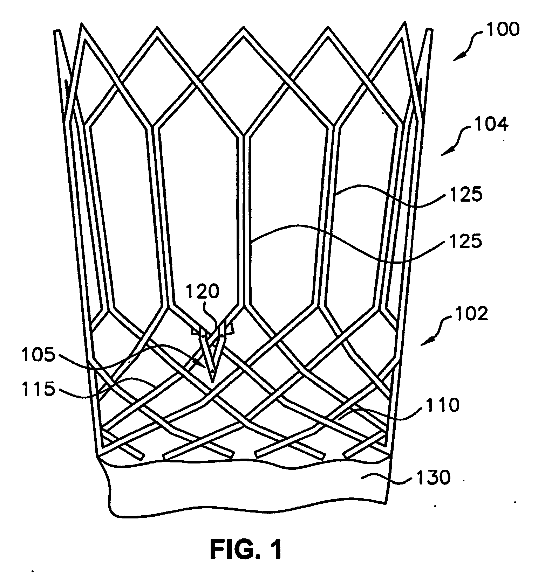

The device of the present invention uses an implant and a barb or barb assembly. The implant used in the present invention can be any number of suitable stents known in the art. A number of suitable stent configurations are described and referenced in co-pending U.S. patent application number 09/442,165, entitled MULTI-SECTION FILAMENTARY ENDOLUMINAL STENT, assigned to the assignee of this application and incorporated herein by reference. The stent may be wound, braided, or made from a laser-cut tube. The stent may be self-expanding or may be capable of expansion by an external force, such as a balloon. The material of the stents may also be any suitable material typically used for such applications, such as nitinol. In the embodiments discussed, the stent has a braided section 102 and a wound section 104, as shown for example in Fig. 1. In the embodiments described, each stent has a radially compressed configuration suitable for loading into an introducer and a radially expanded configuration which it assumes or is caused to assume upon deployment in a body lumen. Also, the stents described herein have a filament, which can be a wire, strand, or a remaining portion from a laser-cut tube.

Fig. 1 depicts a device according to a first embodiment of the present invention. Fig. 1 shows an expanded filamentary stent 100 having a braided section 102 and a would section 104, as is described in the '165 application. Stent 100 comprises a first filament 110 and a second filament 115, both of which extend along both braided section 102 and wound section 104. Within the wound section, a plurality of hexagonal cells 125 (also referred to herein as "vertical cells") are formed by the filaments, with each cell having a base defined by two segments of the hexagonal cell. First filament 110 and second filament 115 also form a plurality of intersections, such as intersection 120, defined by the two filaments crossing one another.

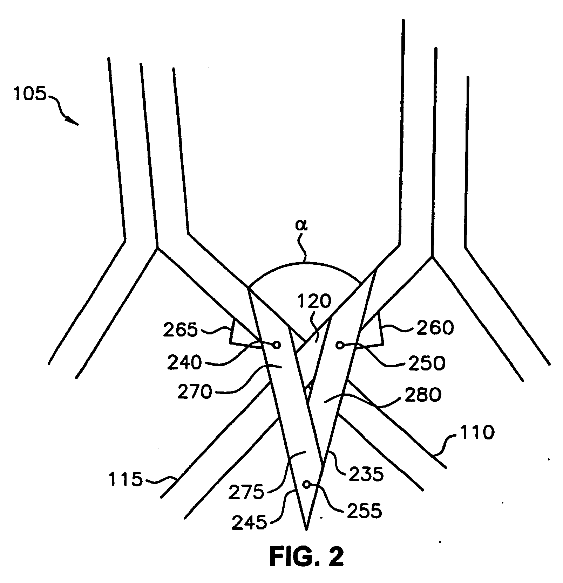

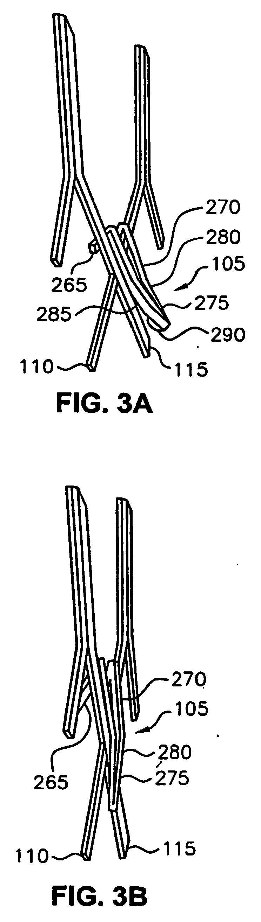

The device shown in Fig. 1 also includes a self-deploying barb assembly 105, which is attached to stent 100 adjacent intersection 120. Figs. 2, 3a, and 3b show self-deploying barb assembly 105 in more detail. As shown therein, self-deploying barb assembly 105 comprises: (i) a first portion 270 attached to the stent, (ii) a bend 280, and (iii) a second portion 275, disposed opposite the first portion from the bend and having a bearing surface 285. Bearing surface 285 is the underside of second portion 275, as viewed in Fig. 2. Barb assembly includes a first wire 235 and a second wire 245, each of which extending across first portion 270 and second portion 280 and each having a bend 275. As shown in these figures, a first end of first wire 235 and a first end of second wire 245 are disposed within first portion 270 and are attached to stent 100. The other ends of the two wires are attached to one another to form a point. More specifically, second wire 245 is attached to first filament 110 and first wire 235 is attached to second filament 115 in the area of intersection 120. A wide variety of ways to attach the wires to the filaments may be employed, e.g. welding, suturing, gluing, and the like, so long as the means for attachment do not adversely affect the biocompatibility of the stent.

Self-deploying barb assembly 105 is pre-fabricated and made of a biocompatible wire, such as nitinol or a material compatible with the biocompatible material of stent 100. In this particular example, self-deploying barb assembly 105 is in the area of intersection 120, which is in a row of stent 100 between braided section 102 and wound section 104. More specifically, barb assembly 105, including bend 120, is disposed adjacent intersection 120. The present invention is not limited to this configuration. Self-deploying barb assemblies 105 may also be fixed to vertical cell segments 125 or to another row within braided section 102. Stent 100 may include a plurality of self-deploying barb assemblies 105 attached along the perimeter of stent 100 and having variable dimensions and geometry, as long as both stent 100 and self-deploying barb assemblies 105 function within a medically acceptable tolerance.

In some embodiments, the device may also include a graft 130 as shown in Fig. 1. Such grafts may be used in an endoluminal device for treating AAA. Grafts serve to prevent blood from flowing across the device to an aneurysm sac. The material for such grafts may be any suitable material used for such purposes, and the graft may be a braided or non-braided graft, and may comprise any graft material known in the art. Suitable graft materials include, but are not limited to, polyethyleneterepthalate (PET), polyetheretherketone (PEEK), polysulfone, polytetrafluroethylene (PTFE), expanded polytetrafluroethylene (ePTFE), fluorinated ethylene propylene (FEP), polycarbonate urethane, a polyolefin (such as polypropylene, polyethylene, or high density polyethylene (HDPE)), silicone, and polyurethane. Preferably, and as shown in Fig. 1, graft 130 is affixed to stent 100 at an area remote from (i.e., axially distant from) barb assembly 105. Typically, the portion where the barbs are located are intended to be placed in the body lumen at a location where there is healthy tissue; on the other hand, a graft is located at a position along the device corresponding to an unhealthy portion of the body lumen, such as an aneurysm sac.

Fig. 2 shows self-deploying barb assembly 105 in more detail including first flat wire 235, a first wire hinge 240, second flat wire 245, a second wire hinge 250, an apex weld 255, a first posterior tab 260, and a second posterior tab 265. Apex weld 255 joins first flat wire 235 to overlapping second flat wire 245, as mentioned above. To prepare the device, self-deploying barb assembly 105 is typically pre-fabricated from a suitable material, such as spring steel, nitinol, or other suitable metals. The assembly is then affixed to first filament 110 and to second filament 115 using first wire hinge 240 and second wire hinge 250, respectively, in the area where first filament 110 and second filament 115 form intersection 120. According to an embodiment of the invention, a first posterior tab 260 and a second posterior tab 265 limit rotation of the hinge on self-deploying barb assembly 105, causing the barb to engage as the diameter of stent 100 changes upon expansion.

Fig. 3a shows a three-dimensional view of a segment of the device of Figs. 1 and 2 including stent 100, comprising first filament 110 and second filament 115, with the device in its radially expanded configuration. Also shown is an engaged barb assembly 105. When the diameter of stent 100 is increased, the forces exerted on barb assembly 105 cause it to flip from a sub-surface profile in a generally outward direction relative to an axis of stent 100 to engage the vessel wall, as discussed in more detail below. As used herein, the term "engage" means when a portion of the barb assembly protrudes into and contacts the body lumen in a way which decreases migration of the device relative to the body lumen.

Fig. 3b is a three-dimensional view of a segment of the device of Figs. 1 and 2 including a stent 100 comprising first filament 110 and second filament 115, and an unengaged self-deploying barb 105. When stent 100 is compressed in the deployment catheter, it is formed to be biased in a radially inward direction relative to an axis of stent 100, and thereby preventing the point of barb assembly 105 from scratching the catheter wall.

As can be seen when comparing Figs. 3a and 3b, second portion 275 of barb assembly 105 (i.e., that portion below the bend 280) swings radially outward to engage the lumen wall as stent 100 radially expands. Thus, second portion 275 is adapted to protrude radially inward when stent 100 is in its radially compressed configuration. This can be done in any number of ways, such as by using a shape memory alloy, such as nitinol which could be configured to have the desired shape in the radially compressed configuration. Spring steel or other metals could also be used. Barb assembly 105 is caused to take its shape as shown in Fig. 3a due to a filament or intersection radially contacting and imparting a radially outward force against bearing surface 285 of the barb assembly 105. More specifically, the radially outward force from stent 100, as it moves from its radially compressed configuration to its radially expanded configuration, is preferably directed somewhere on the bearing surface 285 of second portion 275. To facilitate this extension of barb assembly, it is desirably to cause the force be directed to the end of the second portion furthest from bend 280.

As is known, the angle of some intersections of certain types of stents changes as the stent moves from a radially compressed configuration to a radially expanded configuration. This is true for braided stents or braided portions of stents, such as braided portion 102, in which angle α is shown in Fig. 2. This means that, as stent 100 expands, first filament 110 and second filament 115 swing relative to one another as angle α increases. Thus, the swinging of second filament 115 against bearing surface 285 of second portion 280 can enhance the radial expansion of barb assembly 105 in concert with the radially outward force caused by the expanding stent generally. Preferably, a protuberance 290 is formed on the radially inner side of second portion 280 for abutting against stent 100 as the stent moves between the radially compressed configuration and the radially expanded configuration. Such a protuberance is located at a position such that a filament crosses and contacts the protuberance during radial expansion of the stent.

A method for implanting an endoluminal device in a body lumen involves first compressing the endoluminal device into a radially compressed configuration and retaining it in an introducer. Such an introducer may be a delivery catheter as are well known in the art, such as those described in U.S. Patent Application No. 09/573,273, entitled STENT DELIVERY SYSTEM FOR PREVENTION OF KINKING, AND METHOD OF LOADING AND USING SAME, assigned to the assignee of this application and incorporated herein by reference. Next, the introducer is introduced or threaded into the body lumen via a vascular access site to a deployment location, such as by using a well-known percutaneous cut-down technique referred to above. Examples of the vascular access site include the femoral artery. The access site may be surgically exposed and punctured with, for example, an 18-gauge needle. Then, the device is deployed from the introducer and into the body lumen. This is typically done by first aligning the distal end of the device, then retracting an outer sheath of the introducer. After or upon deployment, the endoluminal device expands to form a radial expanded portion and the at least one filament radially contacts the second portion and imparts a radially outward force against the bearing surface as the implant (e.g., stent) moves from its radially compressed configuration to its radially expanded configuration to cause the second portion to protrude radially outward and engage the body lumen when the stent is in its radially expanded configuration. In the event that the stent is self-expanding, the radial expansion of the stent is caused by the removal of the stent from the introducer. On the other hand, if the stent is not self-expanding, the radial expansion of the stent is caused by expanding a balloon (or some other external source of radially outward force) from within the stent.

The hook(s)/barb(s) can be cut, etched, or attached to the longer wire in any way (facing up, down or both). The barbs can be set on the inner side of the stent for loading and deployment. Then, to deploy the barbs to the outer side post implantation of the device a balloon can be inflated or an inner member dilator/sheath on the delivery system can be advanced in the barb area to push or set the barbs to the outer side of the stent.

In connection with any of the embodiments discussed herein, radiopaque markers may be used in the construction of the attachment means. Such markers assist in deploying, moving or removing the stent since the status of the barb can be determined. Preferably, radiopaque material can be used in the construction of the engagement means, thereby permitting the artisan to further reduce the risk of damage.

In another embodiment of the present invention, the barbs are supported such that during loading into the catheter, in the fully loaded state and during deployment there is no contact between the barbs and the catheter wall. Then, either once the barbed area is exposed or the entire stent-graft system is deployed, the barbs are deployed into place by means such as inflating a balloon or advancing a dilator to push the barbs out into place.

Although illustrated and described herein with reference to certain specific embodiments, the present invention is nevertheless not intended to be limited to the details shown. Rather, various modifications may be made in the details within the scope and range of equivalents of the claims.

## Claims

1. A device for implantation in a body lumen comprising:
an implant 100 having a radially compressed configuration and a radially expanded configuration and comprising at least one filament which pivots as said implant 100 moves between said radially compressed configuration and said radially expanded configuration; and
at least one barb assembly 105
**characterized in that**
said barb assembly 105 comprises: (i) a first portion 270 attached to said implant 100, (ii) a bend 280, and (iii) a second portion 275, disposed opposite said first portion 270 from said bend 280 and having a bearing surface 285, wherein said second portion 275 is adapted to protrude radially inward when said implant 100 is in said radially compressed configuration and said at least one filament radially contacts and imparts a radially outward force against said bearing surface 285 as said implant 100 moves from said radially compressed configuration to said radially expanded configuration to cause said second portion 275 to protrude radially outward when said implant 100 is in said radially expanded configuration.

2. The device of claim 1, wherein:
said implant comprises a stent 100;
said barb assembly 105 comprises a first wire 235 and a second wire 245;
a first end of said first wire 235 and a first end of said second wire 245 are disposed within said first portion 270 and are attached to said stent 100; and
the other end of said first wire 235 is attached to the other end of said second wire 245 to form a point in said second portion 275 which translates radially outward away from said device as said stent 100 radially expands.

3. The device of claim 2, wherein:
said implant comprises a stent 100;
said stent 100 comprises a first filament 110 and a second filament 115;
said first wire 235 is attached to said first filament 110; and
said second wire 245 is attached to said second filament 115.

4. The device of claim 3, wherein:
said stent 100 comprises at least one braided section 102 and at least one wound section 104, which is connected to said braided section 102; and
said barb assembly 105 is attached at a row in said stent 100 between said braided section 102 and said wound section 104.

5. The device of claim 4, wherein:
said wound section 104 defines a plurality of hexagonal cells 125; and
said barb assembly 105 is attached to the base of one of said hexagonal cells 125.

6. The device of claim 1, wherein said barb assembly 105 comprises a protuberance 290 on the radially inner side of said second portion 275 for abutting against said implant 100 as said implant 100 moves between said radially compressed configuration and said radially expanded configuration.

7. The device of claim 1, wherein said implant comprises a stent 100, and said stent 100 comprises a plurality of intersections defined by said first filament 110 crossing said second filament 115 and said barb assembly 105 is disposed adjacent a first of said intersections and said bend 280 is located adjacent said first of said intersections.

8. The device of claim 1 wherein said implant comprises a stent 100, and further comprises a graft 130 affixed to said stent 100 remote from said barb assembly 105.

## Patentansprüche

1. Vorrichtung zur Implantation in ein Körpergefäß, umfassend
ein Implantat (100) mit einer radial verdichteten Konfiguration und einer radial erweiterten Konfiguration und mit wenigstens einem Faden der sich dreht, wenn sich das Implantat (100) zwischen der radial verdichteten Konfiguration und der radial erweiterten Konfiguration bewegt; und
wenigstens einer Widerhakenverbindung (105)
**dadurch gekennzeichnet, dass**
die Widerhakenverbindung (105) einen ersten Abschnitt (270), der an dem Implantat angebracht ist, einen Knick (280) und einen zweiten Abschnitt (275) aufweist, der in Bezug auf den Knick (280) gegenüber dem ersten Abschnitt (270) angeordnet ist und eine Auflagefläche (285) aufweist, wobei der zweite Abschnitt (275) adaptiert ist, radial nach inwärts zu ragen, wenn das Implantat (100) sich in der radial verdichteten Konfiguration befindet, und der wenigstens eine Faden die Auflagefläche (285) radial berührt und auf sie eine nach radial auswärts gerichtete Kraft ausübt, wenn das Implantat (100) sich aus der radial verdichteten Konfiguration in die radial erweiterte Konfiguration bewegt, um den zweiten Abschnitt (275) zu veranlassen, radial nach außen zu ragen, wenn sich das Implantat (100) in der radial erweiterten Konfiguration befindet.

2. Vorrichtung nach Anspruch 1, wobei
das Implantat einen Stent (100) umfasst;
die Widerhakenverbindung (105) einen ersten Draht (235) und einen zweiten Draht (245) aufweist;
ein erstes Ende des ersten Drahts (235) und ein erstes Ende des zweiten Drahts (245) innerhalb des ersten Abschnitts (270) angeordnet sind und an dem Stent (100) befestigt sind; und
das andere Ende des ersten Drahts (235) an dem anderen Ende des zweiten Drahts (245) befestigt ist, um einen Punkt im zweiten Abschnitt (275) zu bilden, der sich radial nach außen, weg von der Vorrichtung bewegt, wenn sich der Stent (100) radial erweitert.

3. Vorrichtung nach Anspruch 2, wobei
das Implantat einen Stent (100) umfasst,
der Stent (100) einen ersten Faden (110) und einen zweiten Faden (115) aufweist;
der erste Draht (235) an dem ersten Faden (110) befestigt ist; und
der zweite Draht (245) an dem zweiten Faden (115) befestigt ist.

4. Vorrichtung nach Anspruch 3, wobei
der Stent (100) wenigsten einen geflochten Abschnitt (102) und einen gewundenen Abschnitt (104), der mit dem geflochtenen Abschnitt (102) verbunden ist, aufweist; und
die Widerhakenverbindung (105) in dem Stent (100) an einer Reihe befestigt ist, die zwischen dem geflochtenen Abschnitt (102) und dem gewundenen Abschnitt (104) liegt.

5. Vorrichtung nach Anspruch 4, wobei
der gewundene Abschnitt eine Vielzahl von hexagonalen Zellen (125) definiert; und
die Widerhakenverbindung (105) an der Basis einer der hexagonalen Zellen (125) befestigt ist.

6. Vorrichtung nach Anspruch 1, wobei die Widerhakenverbindung (105) einen Vorsprung (290) auf der radial inneren Seite des zweiten Abschnitts (275) aufweist, der sich an das Implantat (100) anlehnt, wenn das Implantat (100) sich zwischen der radial verdichteten Konfiguration und der radial erweiterten Konfiguration bewegt.

7. Die Vorrichtung nach Anspruch 1, wobei das Implantat einen Stent (100) aufweist, und dieser Stent (100) eine Vielzahl von Knotenpunkten aufweist, die durch die ersten Fäden (110), die die zweiten Fäden (105) kreuzen, definiert werden, und wobei die Widerhakenverbindung (105) an einem ersten der Knotenpunkte anliegend angeordnet ist, und der Knick (280) neben diesem ersten der Knotenpunkte angeordnet ist.

8. Die Vorrichtung nach Anspruch 1, wobei das Implantat einen Stent (100) und weiterhin ein Transplantat (130) aufweist, das an dem Stent (100) entfernt von der Widerhakenverbindung (105) befestigt ist.

## Revendications

1. Dispositif intraluminal comprenant :
un implant 100 ayant une configuration radialement comprimée et une configuration radialement étendue et comprenant au moins un filament qui pivote tandis que ledit implant 100 se déplace entre ladite configuration radialement compressée et ladite configuration radialement étendue ; et
au moins un ensemble 105 de barbes
**caractérisé en ce que**
ledit ensemble 105 de barbes comprend : (i) une première partie 270 attachée audit implant 100, (ii) un pli 280, et (iii) une deuxième partie 275, disposée à l'opposé de ladite première partie 270 à partir dudit pli 280 et ayant une surface 285 d'appui, dans laquelle la deuxième partie 275 est adaptée pour dépasser vers l'intérieur radialement lorsque ledit implant 100 est dans ladite configuration radialement comprimée et que ledit au-moins un filament entre radialement en contact et imprime vers l'extérieur radialement une force contre ladite surface 285 d'appui tandis que ledit implant 100 se déplace de ladite configuration radialement comprimée vers ladite configuration radialement étendue pour entraîner ladite deuxième partie 275 à dépasser vers l'extérieur radialement lorsque ledit implant 100 se trouve dans ladite configuration radialement étendue.

2. Dispositif selon la revendication 1, dans lequel :
ledit implant comprend une endoprothèse vasculaire 100 ;
ledit ensemble 105 de barbes comprend un premier fil 235 et un deuxième fil 245 ;
une première extrémité dudit premier fil 235 et une première extrémité dudit deuxième fil 245 sont disposées à l'intérieur de ladite première partie 270 et sont attachées à ladite endoprothèse vasculaire 100 ; et
l'autre extrémité dudit premier fil 235 est attachée à l'autre extrémité dudit deuxième fil 245 pour former un point dans ladite deuxième partie 275 qui effectue une translation radialement vers l'extérieur loin dudit dispositif tandis que ladite endoprothèse vasculaire s'étend radialement.

3. Dispositif selon la revendication 2, dans lequel :
ledit implant comprend une endoprothèse vasculaire 100 ;
ladite endoprothèse vasculaire 100 comprend un premier filament 110 et un deuxième filament 115 ;
ledit premier fil 235 est attaché audit premier filament 110 ; et
ledit deuxième fil 245 est attaché audit deuxième filament 115.

4. Dispositif selon la revendication 3, dans lequel :
ladite endoprothèse vasculaire 100 comprend au moins une section 102 tressée et au moins une section 104 enroulée, qui est reliée à ladite section 102 tressée ; et
ledit ensemble 105 de barbes est attaché à une rangée dans ladite endoprothèse vasculaire 100 entre ladite section 102 tressée et ladite section 105 enroulée.

5. Dispositif selon la revendication 4, dans lequel :
ladite section 104 enroulée définit une pluralité de cellules 125 hexagonales ; et
ledit ensemble 105 de barbes est attaché à la base d'une des cellules hexagonales 125.

6. Dispositif selon la revendication 1, dans lequel ledit ensemble 105 de barbes comprend une protubérance 290 sur le côté radialement interne de ladite deuxième partie 275 pour prendre appui sur ledit implant 100 tandis que ledit implant 100 se déplace entre ladite configuration radialement comprimée et ladite configuration radialement étendue.

7. Dispositif selon la revendication 1, dans lequel ledit implant comprend une endoprothèse vasculaire 100, et ladite endoprothèse vasculaire 100 comprend une pluralité d'intersections définies par ledit premier filament 110 qui croise ledit deuxième filament 115 et ledit ensemble 105 de barbes est disposé adjacent à la première desdites intersections et ledit pli 280 est situé adjacent à ladite première desdites intersections.

8. Dispositif selon la revendication 1 dans lequel ledit implant comprend une endoprothèse vasculaire 100, et comprend en outre un greffon 130 fixé à ladite endoprothèse vasculaire 100 éloigné dudit ensemble 105 de barbes.
